# EUROPEAN PATENT APPLICATION

(11) **EP 2 735 989 A1**
(43) Date of publication of application: **28.05.2014**
(21) Application number: 12193769.2
(22) Date of filing: 22.11.2012
(51) Int. Cl.: G06F 19/00

(54) **Method and computer program for managing measurements on medical images**

(71) Applicant: Agfa Healthcare, 2640 Mortsel (BE)
(72) Inventor: Bogaert, Caroline, 2640 Mortsel (BE); Kieckens, Wannes, 2640 Mortsel (BE); Felix, Joost, 2640 Mortsel (BE)

(57) **Abstract**

There is provided a method of operating a data processing system (1) for managing measurements on medical images. This method automatically updates measurement information (30) in a draft medical report (40) when the measurement (10) on the medical image (20) is modified

## Description

### Field of the Invention

The present invention generally relates to management of the measurements to be performed by an individual or a team of individuals in a medical imaging application. Measurement management is important especially when involving medical imaging applications, where for example a technician performs measurements on medical images of a patient (e.g. an x-ray scan, a CT scan, an MRI scan, an ultrasound scan, etc.), a radiologist interprets these measurements on the medical images and produces a report reflecting his interpretation of the measurements, and a physician receives the report and advises the patient. The individuals involved in the measurement workflow may be working at a single facility (e.g. a hospital or imaging centre) or may be working at different locations (e.g. a hospital or enterprise with multiple facilities). In order to govern the different measurements to be performed, a measurement management tool will be deployed and made accessible to the involved.

### Background of the Invention

The increasing trend of evidence based practice in medical imaging requires medical imaging professionals to support their reported findings with quantitative information. Historically many findings were purely described in a qualitative manner. There is now a tendency for medical imaging professionals to quantify their findings before qualifying them. Additionally recent developments in medical imaging technology allow for efficient acquisition of quantitative measurement data in addition to the medical image data, such as for example Magnetic Resonance spectrometry, fluid streams in ultrasound examinations, diffusion and perfusion measurements on Computed Tomography and Magnetic Resonance data, metabolic measurements on information from nuclear medicine images, etc. These measurements are an important part of the diagnostic process as they play a crucial role in the diagnosis, treatment and follow up of the patient. Technologists, sonographers, radiologists, orthopedic surgeons, etc. regularly perform measurements as part of the normal image acquisition protocols. Examples are measurements of the evolution of the size of an aneurysm during a plurality of follow-up examinations, the percentage of vessel stenosis as an indication for an intervention, the growth or decline of metabolic activities of suspicious lesions, the determination of the size of a prosthesis prior to the intervention, etc. In addition to support treatment and follow-up during patient care, a plurality of measurements from image acquisitions taken at a plurality of instances in time also frequently need to be compared. In oncology, for example, it is important to be able to determine the rate of growth or decline of a tumor in order to verify the efficacy of an on-going therapy, alternatively it can support any choices to be made during the treatment plan by comparing the measured values to for example a statistically relevant normal value. Such measurements preferably need to be tracked in a long term medical record and are thus an important part of medical reports that are generated.

It is known from for example W02006/008234 to provide a report generator that automatically includes graphical measurements and calculations thereon into a structured report in order to avoid human errors. It is further known from EP1349098 to provide a system that is able to assist in performing measurements and is able to perform calculations on graphical measurements by means of visualisation and comparison with normal values for these measurements. However in these prior art systems report generation the graphical measurements are merely added to the report in a final step of a workflow. In order to take into account a plurality of measurements taken at different instances in time, a plurality of such reports must be reviewed and often comparative information must be calculated manually.

Thus there still exists a need for more efficient and flexible creation of reports comprising graphical measurements and calculations.

### Summary of the Invention

According to a first aspect of the invention there is provided a method of operating a data processing system for managing measurements on medical images, said method comprising the steps of:
- performing a measurement on a medical image;
- automatically providing a draft medical report comprising measurement information calculated in function of said measurement;
- automatically updating said measurement information in said draft medical report when said measurement on said medical image is modified.

This allows for synchronisation of the measurements on the medical image and the information in the draft medical report. This is especially useful when the measurements and the draft report are iteratively adjusted by different actors in the workflow. In this way human errors are avoided which are for example caused by discrepancies between the measurements on the medical image and those available in the medical report which could lead to a faulty diagnosis. It further also increases the efficiency with which the medical report can be created as measurement data is automatically available during the drafting of the medical report in support of the assessments to be provided in the report.

According to an embodiment said method comprises the further step of:
- retrieving a stored measurement related to said measurement;
- automatically providing said draft medical report further comprising stored measurement information calculated in function of said stored measurement.

In this way stored measurements, for example of previous measurements during a treatment plan, can be added to the draft report efficiently in order to support a diagnosis for which an interpretation of a plurality of measurements is necessary, such as for example the growth rate of a tumor.

According to a further embodiment said stored measurement comprises a previous measurement on a previous medical image.

This allows to efficiently analyse and report an evolution of these measurements in time.

According to still a further embodiment said stored measurement comprises a statistical reference measurement.

This additionally allows to compare these measurements with for example normal values for a given reference population.

According to still a further embodiment said method comprises the further step of automatically providing said draft medical report further comprising comparative measurement information calculated in function of both said measurement and said stored measurement.

In this way the draft report can be created efficiently and document a diagnosis that is based on comparative measurement information objectively.

According to a further embodiment said method comprises the further step of associating said comparative measurement information with a label selected from a plurality of predetermined labels associated with a corresponding plurality of predetermined ranges for said comparative measurement information.

This allows to automate diagnosis and generation of the medical report by categorizing the comparative measurement information which allows for a more uniform interpretation of the measurement information. When comparative measurement information indicates a growth percentage of a detected tumor above a predetermined growth percentage, this could for example be linked to a label that signals active tumor growth.

According to a preferred embodiment said draft medical report comprises instructions to automatically adapt the contents of said draft medical report in function of said comparative measurement information.

This still further automates the creation of the medical report. For example based on the comparative measurement information it could be automatically determined whether there was a significant change which indicates a predetermined assessment such as for example whether or not a specific event such as an infarction has occurred or for example a preferred dosage regimen for medicaments could be calculated.

According to a further embodiment said measurement is selected from a measurement list, comprising a plurality of predetermined types of measurements.

According to a further embodiment said draft medical report is selected from a medical report list, comprising a plurality of predetermined medical report templates.

According to a second aspect of the invention the data processing system comprising means for carrying out the method according to the first aspect of the invention.

According to a third aspect of the invention there is provided a computer program comprising software code adapted to perform the method according to the first aspect of the invention.

According to a fourth aspect of the invention there is provided a computer readable storage medium comprising the computer program of according to the first aspect of the invention.

### Brief Description of the Drawings

Fig. 1 schematically illustrates an embodiment of the method according to the invention;

Fig. 2 schematically illustrates a suitable computing system for operating according to the method of Fig. 1.

### Detailed Description of Embodiment(s)

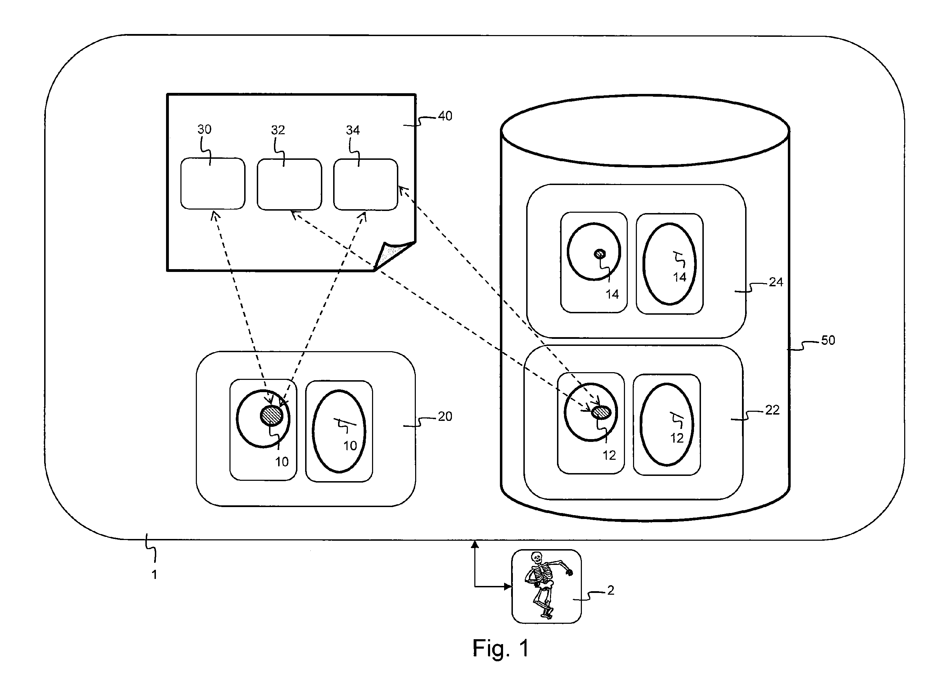

Figure 1 shows schematically the method of operation of a data processing system 1 for managing measurements on medical images. Such a system is for example implemented as a software application comprising suitable programming instructions for execution by a processor of a computing system. Alternatively it could form a component of a software application, for example in the form of an application module, by means of a programming library, as network component being accessible through a suitable application program interface, or any other suitable implementation. The system 1 is especially beneficial when being part of a system that automatically generates a large number of workflows that comprise interrelated tasks for a large number of actors. This is for example the case in the context of a workflow management system in a medical imaging environment, such software application is for instance important in medical imaging applications, where a nurse needs to suitably prepare the patient for the imaging operation by providing and recording information to and from the patient, a technician acquires medical images of a patient (e.g. an x-ray scan, a CT scan, an MRI scan, an ultrasound scan, etc.) and post-processes these images in order to enable specific forms of analysis, a radiologist interprets the medical images and produces a report reflecting his interpretation, and a physician receives the report and advises the patient. The individuals involved in the entire workflow may be working at a single facility (e.g. a hospital or imaging centre) or may be working at different locations (e.g. a hospital or enterprise with multiple facilities). The workflow management system is preferably connected to an image acquisition device 2 in order to automatically communicate with such a device 2, for example via a suitable network interface. In this way it is able to receive image data and optionally other data such as for example measurement data from measurements performed on the medical images provided by the medical image acquisition device 2.

When performing a measurement 10 on a medical image 20, the user of the system 1 usually performs the measurement by graphically indicating an item of interest on the medical image 20. The medical image 20 represented in Figure 1 schematically illustrates a front view and a side view of a CT scan. In the top view the user performs a measurement 10 by drawing a circle around an area of interest in the top view, for example representative of a tumor. According to this embodiment the system will automatically also indicate the measurement 10 as a corresponding line in the corresponding side view, where it is visualised as a line. In order to perform the measurement the user preferably selects the graphical measurement from a measurement list, comprising a plurality of predetermined types of measurements which can subsequently be drawn on the medical image 20. Such types of graphical measurements comprise for example an angle, an arrow, a circle, spine labeling, a Cobb angle, a hip-knee-ankle angle, a sphere, a cylinder, a curve, etc. It is further clear that instead of a single measurement performed on the medical image, a plurality of such measurements could be performed on a medical image 20. This medical image 20 could be a processed, rendered or native image or group of images derived from the image data provided by the image acquisition device 2.

The system 1 will then calculate measurement information 30 in function of this measurement 10. For example for the circle shown in the embodiment of Figure 1 this measurement information 30 could for example comprise one or more properties of the circle such as for example the area, circumference, diameter, mean value, etc. The user is able to select which of these properties are to be provided as measurement information. This measurement information can then optionally be displayed along with the graphical measurement shown on the medical image 20. Further the system 1 will automatically provide a draft medical report 40 comprising this measurement information 30. After a user has performed an initial measurement on the medical image by for example position the circle around an area of interest, the user or another user will still be able to adjust this measurement by for example repositioning or resizing the measurement. When the measurement 10 on the medical image 20 is modified the system will automatically update the measurement information 30. In this way the measurement information 30 in the draft medical report 40 and optionally also on the medical image 20 will be recalculated automatically and will remain synchronised with any modifications during the entire workflow associated with the medical image 20 until the final step in the workflow is reached in which the draft report is finalized, for example by signing it of by the responsible physician, after which further modification is disabled.

According to the embodiment shown in Figure 1, the system 1 is configured to retrieve stored measurements 12, 14, which are related to the measurement 10 performed on a medical image. When for example the measurement 10 as illustrate concerns the measurement of several properties of a brain tumor in a CT scan, it could for example concern a previous measurement 12, 14 of the same properties performed during a previous CT scan of the same patient and stored in a suitable storage structure 50 such as for example a suitable database. Such a stored measurement 14 will then also be provided to the draft medical report 40 by the system 1 in the form of stored measurement information 32 which comprises properties that are calculated in function of this stored measurement 12. This assists the user of the system 1 to draft other parts of the draft report efficiently taking into account the measurement information 30 which can immediately be compared with the stored measurement information 32, 34. Preferably, as shown in the embodiment of Figure 1, in order to further assist the user in qualifying the comparison the measurement 10 with the stored measurement 12, 14, the system 1 will automatically calculate comparative measurement information 34. If the measurement information 30 and the previous measurement information 32 comprises for example the area of tumor at two different instances in time, such comparative measurement information 34, could for example be a calculated percentage of increase or decrease of this area, indicating the degree of tumor growth or decline. Such comparative measurement information 34 calculated in function of both the measurement 10 and the stored measurement 12, 14 is then also automatically provided by the system 1 to the draft medical report 40 in order to still further augment the efficiency of drafting it and clearly quantifying the assessments provided therein.

Further according to the embodiment of Figure 1 the stored measurement 32 could also comprise a statistical reference measurement 14. Such a statistical reference measurement 14 could be selected from a statistical reference library 24 that for example provides statistical reference measurements 14 that are selectable in function of the measurement 10 being performed by for example providing a mean value for such a measurement 10 for a relevant healthy population as for example determined by the age, gender and medical history of the patient or any other suitable parameters. This information will provide the user of the system 1 drafting the draft medical report 40 with additional information for supporting and documenting the assessments made in it. Still a further step in automation can be achieved by associating the comparative measurement information 34 with a label. Such a label is selected from a plurality of predetermined labels, such as for example low, medium, high, associated with a corresponding plurality of predetermined ranges for the comparative measurement information 34 based on a measurement 10 and one or more previous measurements 12, such as for example an increase of the area of a tumor with respectively less than 5%, between 5% and 10% and more than 10%. Alternatively such labels could for example be normal, abnormal and correspond to two respective ranges of comparative measurement information 34 based on a statistically significant or insignificant difference between a measurement 10 and a relevant statistical reference measurement 14. It is clear that a variety of alternative labels and corresponding ranges could be provided. Such a label gives an easy and intuitive feedback to the healthcare professional and allows for a more uniform assessment to be provided irrespective of the specific user drafting the draft medical report 40. According to an embodiment with still a further level of automation the draft medical report 40 comprises instructions to automatically adapt the contents of the draft medical report 40 in function of the comparative measurement information 34. In this way in addition to merely providing labels associated with specific ranges, standard phrases describing the assessment could be automatically inserted into the draft report based on such ranges.

In order to optimise the creation of the draft medical report 40 it can preferably be selected by the user from a medical report list. Such a medical report list comprises a plurality of predetermined medical report templates. These report templates can be configured upfront by the user and comprises instructions on how to insert the measurement information 30 and optionally stored measurement information 32 or comparative measurement information 34 into the draft medical report 40. Such report templates for example define the properties that are calculated from the measurement 10, the algorithm for such calculations and the position and formatting of this measurement information 30 in the medical report. Each of these templates can be selected in function of parameters such as for example one or more medical examination types, modality types or pathologies.

According to an alternative embodiment instead of the report templates the measurement information 30 and optionally stored measurement information 32 or comparative measurement information 34 could be inserted into the draft medical report 40 by means of text macros. Such text macros equally comprise the necessary instructions to calculate the measurement information 30 from the measurement 10 and insert it automatically into the draft medical report 40. Each of these text macros can also be selected in function of parameters such as for example one or more medical examination types, modality types or pathologies. Such text macros are predefined texts wherein predetermined fields are foreseen to insert specific measurement information. The text macro further also comprises all necessary instructions to perform the desired calculations on this measurement information when such a text macro is inserted into the draft medical report 40 by the user. The predetermined fields are provided with measurement information calculated from the measurement performed on the medical image and any calculations are automatically performed as soon as all necessary measurements are available.

It is clear that the measurements 10 that are automatically included in the medical draft report 40 are not restricted to measurements 10 that are generated by means of the system 1. According to an alternative a technologist could perform measurements 10 on the image acquisition device 2 that acquired the medical images 20. Such measurements 20 can then be provided to the system 1 alongside with the image data in the form of measurement data by means of a suitable communication link.

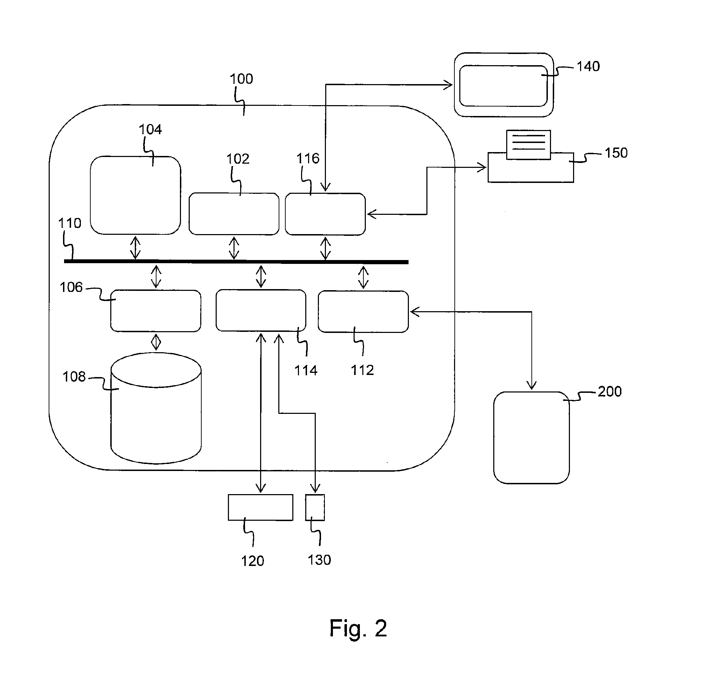

Figure 2 shows a suitable computing system 100 for hosting data processing system 1 of Figure 1. Computing system 100 may in general be formed as a suitable general purpose computer, such as a workstation, a server, a laptop, a desktop, a hand-held device, a mobile device, a tablet computer, or other computing device, as would be understood by those of skill in the art. The computing system 100 comprises a bus 110, a processor 102, a local memory 104, one or more optional input interfaces 114, one or more optional output interfaces 116, a communication interface 112, a storage element interface 106 and one or more storage elements 108. Bus 110 may comprise one or more conductors that permit communication among the components of the computing system. Processor 102 may include any type of conventional processor or microprocessor that interprets and executes programming instructions. Local memory 104 may include a random access memory (RAM) or another type of dynamic storage device that stores information and instructions for execution by processor 102 and/or a read only memory (ROM) or another type of static storage device that stores static information and instructions for use by processor 102. Input interface 114 may comprise one or more conventional mechanisms that permit an operator to input information to the computing device 100, such as a keyboard 120, a mouse 130, a pen, voice recognition and/or biometric mechanisms, etc. Output interface 116 may comprise one or more conventional mechanisms that output information to the operator, such as a display 140, a printer 150, a speaker, etc. Communication interface 112 may comprise any transceiver-like mechanism such as for example two 1 Gb Ethernet interfaces that enables computing system 100 to communicate with other devices and/or systems, for example mechanisms for communicating with one or more other computing systems 200. The communication interface 112 of computing system 100 may be connected to such another computing system by means of a local area network (LAN) or a wide area network (WAN, such as for example the internet, in which case the other computing system 200 may for example comprise a suitable web server. Storage element interface 106 may comprise a storage interface such as for example a Serial Advanced Technology Attachment (SATA) interface or a Small Computer System Interface (SCSI) for connecting bus 110 to one or more storage elements 108, such as one or more local disks, for example 1TB SATA disk drives, and control the reading and writing of data to and/or from these storage elements 108. Although the storage elements 108 above is described as a local disk, in general any other suitable computer-readable media such as a removable magnetic disk, optical storage media such as a CD or DVD disk, solid state drives, flash memory, Random Access Memory (RAM), Read Only Memory (ROM), Electronically Erasable Programmable Read Only Memory (EEPROM) or other memory technologies, holographic media, magnetic cassettes, magnetic tape, magnetic disk storage or other magnetic storage devices that can be used to encode information and can be accessed by the computing system 100 could be used.

The data processing system 1 can be implemented as programming instructions stored it local memory 104 of the computing system 100 for execution by its processor 102. Alternatively the data processing system 1 could be stored on the storage element 108 or be accessible from another computing system 200 through the communication interface 112. Such a plurality of suitably connected computing systems could for example form a system or platform with client-server architecture in which the medical images, reports and tasks are maintained centrally or distributed on one or more servers.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A method of operating a data processing system (1) for managing measurements on medical images, said method comprising the steps of:
- performing a measurement (10) on a medical image (20);
- automatically providing a draft medical report (40) comprising measurement information (30) calculated in function of said measurement (10);
- automatically updating said measurement information (30) in said draft medical report (40) when said measurement (10) on said medical image (20) is modified.

2. A method according to claim 1, **characterised in that** said method comprises the further step of:
- retrieving a stored measurement (12, 14) related to said measurement (10);
- automatically providing said draft medical report (40) further comprising stored measurement information (32) calculated in function of said stored measurement (12).

3. A method according to claim 1 or 2, **characterised in that** said stored measurement (32) comprises a previous measurement (12) on a previous medical image (22).

4. A method according to any of the preceding claims, **characterised in that** said stored measurement (32) comprises a statistical reference measurement (14).

5. A method according to any of the claims 2 to 4, **characterised in that** said method comprises the further step of automatically providing said draft medical report (40) further comprising comparative measurement information (34) calculated in function of both said measurement (10) and said stored measurement (12, 14).

6. A method according to claim 5, **characterised in that** said method comprises the further step of associating said comparative measurement information (34) with a label selected from a plurality of predetermined labels associated with a corresponding plurality of predetermined ranges for said comparative measurement information (34).

7. A method according to claim 5 or 6, **characterised in that** said draft medical report comprises instructions to automatically adapt the contents of said draft medical report (40) in function of said comparative measurement information (34).

8. A method according to any of the preceding claims, **characterised in that** said measurement (10) is selected from a measurement list, comprising a plurality of predetermined types of measurements.

9. A method according to any of the preceding claims, **characterised in that** said draft medical report (40) is selected from a medical report list, comprising a plurality of predetermined medical report templates.

10. A data processing system comprising means for carrying out the method of any of claims 1 to 9.

11. A computer program comprising software code adapted to perform the method of any of claims 1 to 9.

12. A computer readable storage medium comprising the computer program of claim 11.
